# EUROPEAN PATENT SPECIFICATION

(11) **EP 3 431 066 B1**
(45) Date of publication and mention of the grant of the patent: **16.12.2020**
(21) Application number: 18184040.6
(22) Date of filing: 17.07.2018
(51) Int. Cl.: A61G 7/057, A61G 7/015, A61G 7/07

(54) **PATIENT COOLING SYSTEM RESPONSIVE TO HEAD ELEVATION**
AUF DIE KOPFERHEBUNG REAGIERENDES PATIENTENKÜHLSYSTEM
SYSTÈME DE REFROIDISSEMENT DE PATIENT RÉAGISSANT À UNE ÉLÉVATION DE LA TÊTE

(30) Priority: 21.07.2017 US 201762535324 P
(43) Date of publication of application: 23.01.2019
(73) Proprietor: Hill-Rom Services, Inc., Batesville, IN 47006-9167 (US)
(72) Inventor: LACHENBRUCH, Charles, A, Batesville, IN Indiana 47006-9167 (US); WILLIAMS, Joshua, A, Batesville, IN Indiana 47006-9167 (US)
(74) Representative: Findlay, Alice Rosemary

(56) References cited:
- US-B1- 9 226 863

## Description

The present disclosure is related to bed mattresses for supporting patients. More specifically, the present disclosure is related to a microclimate structure for hospital beds, medical beds, or other types of beds in which the microclimate structures are designed to cool and dry a patient's skin around targeted therapeutic regions.

In a care facility, such as a hospital or a nursing home, patients are often placed on patient support apparatuses for an extended period of time. Patients who are positioned on the patient support apparatus often have a risk of developing certain skin condition, such as bed sores (also known as pressure sores or decubitus ulcers), due to heat and moisture along the interface of the patient with the surface of the bed mattress. In an effort to mitigate or prevent such conditions, some bed mattresses have a built-in microclimate structure. The microclimate structure may conduct air along the interface of a patient with the surface to keep the patient's skin cool and dry. Some microclimate structures require a large volume of air to be supplied to them in order to provide an effective amount of cooling and drying to a patient's skin.

US 9,226,863 B1 describes a mattress for relieving pressure ulcers. The mattress includes a first sponge layer, an air mattress layer over the first sponge layer, an intermediate layer over the air mattress layer, a second sponge layer over the intermediate layer, and a paper sheet layer over the second sponge layer. The sponge layer and the air mattress layer are configured to increase the contact area of the patient's body with the bed, according to a patient's weight. The intermediate layer is a rubber layer configured to provide ventilation for areas of the mattress that contact a patient's body using dry air or dry air mixed with ozone gas, essential/volatile oil, and/or antibacterial vapors. The second sponge layer is designed to distribute the air under the patient's body. The paper sheet layer is configured to provide alarms in the event the mattress gets wet due to sweating or incontinence.

The present invention is defined by the claims.

According to one aspect of the disclosure, a patient support apparatus includes a frame and an air box. A patient support structure is supported by the frame and includes a head section, a foot section, and a seat section between the head section and foot section. The patient support structure includes a cushion layer and an outer ticking layer including an upper surface portion positioned to support a patient. A microclimate structure is positioned within the outer ticking layer and between the cushion layer and the upper surface portion. The microclimate structure includes an upper layer. At least a portion of the upper layer is vapor and liquid permeable. A middle layer is air permeable. A lower layer is liquid impermeable. A first plurality of perforations extends through the upper layer of the microclimate structure in the seat section of the patient support structure. A second plurality of perforations extends through the upper layer of the microclimate structure in the head section of the patient support structure. When the patient support structure is in a first position, the air box supplies air flow through the first plurality of perforations and the second plurality of perforations. When the patient support structure is in a second position, the air box supplies air flow through the first plurality of perforations and air flow through the second plurality of perforations is limited relative to the air flow through the first plurality of perforations and air flow through the second plurality of perforations when the patient support structure is in the first position. When the patient support structure is in the second position, a crease is formed in the microclimate structure between the first plurality of perforations and the second plurality of perforations, and the air flow through the second plurality of perforations is limited by the crease. The middle layer has a higher density or a lower density where the crease is formed, so as to facilitate forming of the crease when the patient support structure is moved to the second position.

In some embodiments, when in the first position, the head section may be declined. In some embodiments, when in the second position, the head section may be inclined. In some embodiments, the microclimate structure may extend from a lower end of the head section to a lower end of the seat section of the patient support structure, excluding the foot section of the patient support structure. In some embodiments, the air box may be coupled to a conduit to conduct pressurized air through the microclimate structure.

In some embodiments, the vapor and liquid permeable portion of the upper layer of the microclimate structure may define a therapeutic region. In some embodiments, the therapeutic region of the upper layer of the microclimate structure may have a highly breathable, vapor and liquid permeable material. In some embodiments, a non-therapeutic region of the upper layer of the microclimate structure may have a vapor permeable but liquid impermeable material. In some embodiments, the middle layer of the microclimate structure may have a three-dimensional material configured to conduct air between the upper layer and the lower layer of the microclimate structure. In some embodiments, the middle layer of the microclimate structure may have more than one section of the three dimensional material, in which structure may have more than one section of the three dimensional material, in which at least one section of the three dimensional material conducts and delivers air along a therapeutic region.

In some embodiments, the foot section of the microclimate structure may be a foam padding. In some embodiments, the cushion layer may have a first inflatable support bladder and a second inflatable support bladder, and an air distribution sleeve that extends between the first inflatable support bladder and the second inflatable support bladder. In some embodiments, the cushion layer may have foam paddings. In some embodiments, the outer ticking layer may be a vapor permeable and liquid impermeable material. In some embodiments, the outer ticking layer may encase the microclimate structure. In some embodiments, the outer ticking layer may encase the microclimate structure and the cushion layer.

According to another aspect of the disclosure, a patient support structure includes a cushion layer. A microclimate structure is integrated atop the cushion layer. The microclimate structure includes an upper layer having a vapor and liquid permeable therapeutic region, an air permeable middle layer, and a liquid impermeable lower layer. A first plurality of perforations extends through the upper layer of the microclimate structure in a seat section of the microclimate structure. A second plurality of perforations extends through the upper layer of the microclimate structure in a head section of the microclimate structure. When the microclimate structure is in a first position, air is supplied through the first plurality of perforations and the second plurality of perforations. When the microclimate structure is in a second position, air is supplied through the first plurality of perforations and air flow through the second plurality of perforations is limited.

In some embodiments, when in the second position, a crease may be formed in the microclimate structure between the first plurality of perforations and the second plurality of perforations. In some embodiments, the crease formed in the microclimate structure may limit the air flow to the second plurality of perforations. In some embodiments, when in the first position, the head section may be declined. In some embodiments, when in the second position, the head section may be inclined.

In some embodiments, a therapeutic region of the microclimate structure may include a highly breathable, vapor and liquid permeable material. In some embodiments, the middle layer of the microclimate structure may include a three-dimensional material configured to conduct air between the upper layer and the lower layer of the microclimate structure. In some embodiments, the middle layer of the microclimate structure may include more than one section of the three dimensional material. At least one section of the three dimensional material may conduct and deliver air along a therapeutic region.

The invention will now be further described by way of example with reference to the accompanying drawings, in which:
Fig. 1 is a perspective view from a foot end on the patient's right of a patient support apparatus including an air box and a patient support structure supported on a frame;
Fig. 2 is a top plan view of the patient support apparatus of Fig. 1 where a targeted therapeutic region extends from a head section through a seat section of the patient support structure, covering an entire upper surface of the microclimate structure;
Fig. 3 is a cross section taken along section lines 3-3 of Fig. 2 showing an embodiment of the patient support structure encased by an outer ticking layer where an upper ticking covers the microclimate structure and a lower ticking encases a cushion layer;
Fig. 4 is a perspective view of the patient support structure having a head end in an inclined position; and
Fig. 5 is a side elevation view of the patient support structure having a head end in an inclined position.

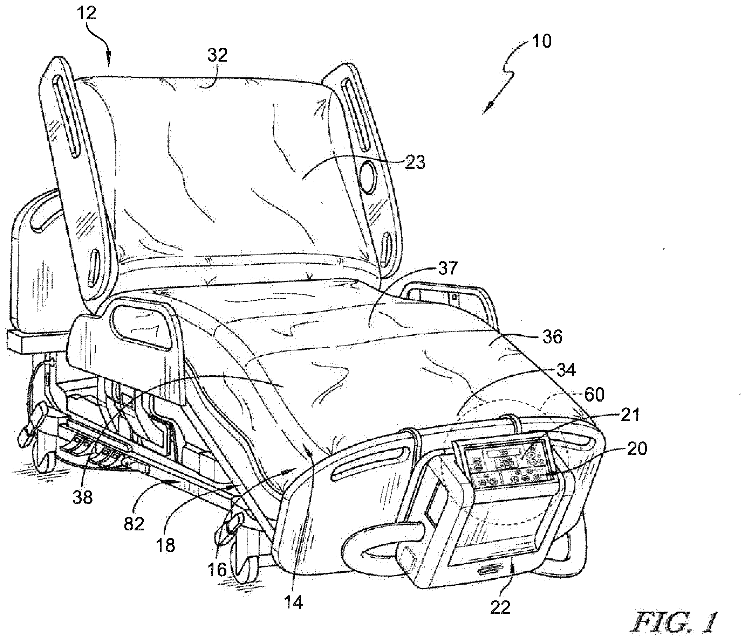

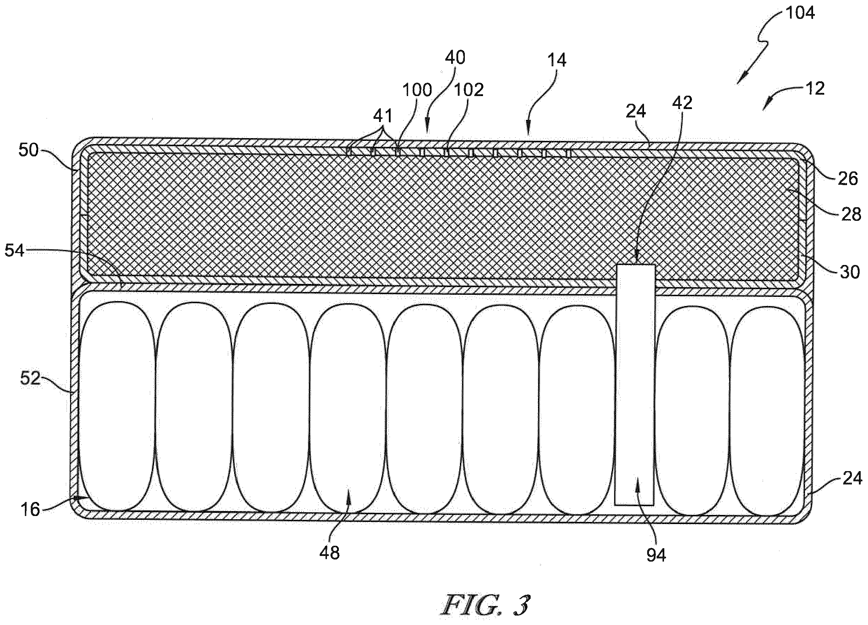

An illustrative patient support apparatus 10 embodied as a hospital bed is shown in Fig. 1. The patient support apparatus 10 includes a frame 18, a patient support structure 12 supported on the frame 18, and an air box 22. The patient support structure 12 is adapted to support a patient lying on the patient support apparatus 10 and includes a head section 32, a seat section 37, and a foot section 34. As will be discussed in further detail below, the patient support structure 12 further includes a microclimate structure 14 and a cushion layer 16 which supports the microclimate structure 14 as shown in Fig. 3. The cushion layer 16 may include a plurality of inflatable support bladders 48. The microclimate structure 14 is positioned on the cushion layer 16 on an occupant side and adjacent a support surface 23 and is configured to conduct air adjacent the support surface 23 of the patient support structure 12. The air conducted by the microclimate structure 14 is pressurized and pushed through the microclimate structure 14 by the air box 22. By conducting air along an interface of the support surface 23 and the patient, the microclimate structure 14 transfers heat and moisture from the patient and cools and dries the patient's skin in order to reduce the risk of bed sore formation by the patient.

The air box 22 further includes a user interface 60 that is configured to receive user inputs. The user interface 60 includes a display screen 21 and a plurality of buttons 20 for inputting patient information and for controlling operation of the air box 22 and the support surface 23. Particularly, the user interface 60 allows a user to adjust the flow of air provided by the air box 22 to the microclimate structure 14 and, in some embodiments, to adjust the temperature of air provided by the air box 22 to the microclimate structure 14. Specifically, in some embodiments, the user interface 60 may include a patient information input panel, an alarm panel, a lateral rotation therapy panel, an inflation mode panel, a normal inflation control panel, and a microclimate control panel.

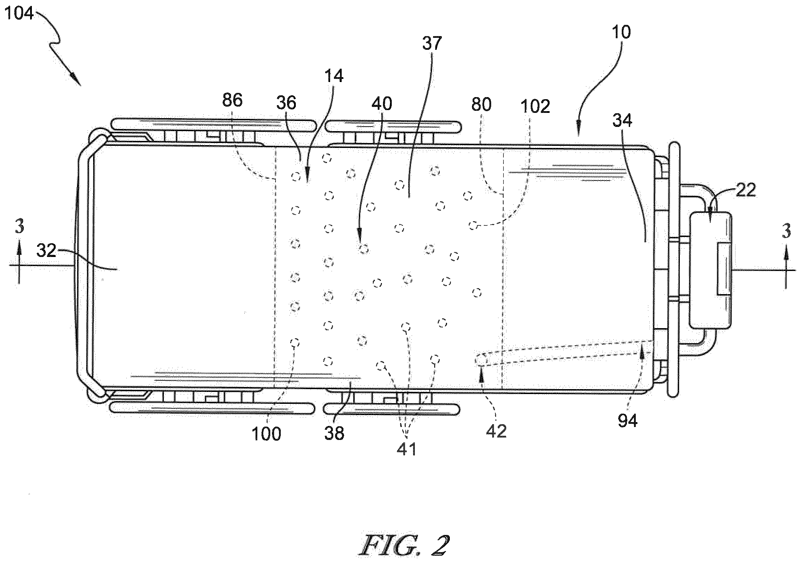

The microclimate structure 14 is configured to receive pressurized air from the air box 22 and to conduct air through the microclimate structure 14 to cool and dry the interface between a patient and the patient support apparatus 10 to promote skin health by removing patient heat and moisture along the interface when the patient is supported on the patient support apparatus 10. The microclimate structure 14 generally spans laterally from a left side 36 to a right side 38 and extends longitudinally from above a lower end 86 of the head section 32 to a lower end 80 of the seat section 37, excluding the foot section 34 of the patient support structure 12 as shown in Fig. 2.

Referring to Fig. 2, in one embodiment, the microclimate structure 14 further includes a therapeutic region 40 which is specifically configured to target specific areas of the patient's body where local climate control is most needed. This corresponds to the areas where the pressure of patient's weight against the support surface 23 is the greatest when the patient is lying supine and centered on the microclimate structure 14. The therapeutic region 40 may be made from a highly breathable material or a perforated material. Because the patient's sweat glands are distributed non-uniformly throughout the patient's body, perspiration tends to accumulate on the skin of the patient's torso and pelvic region. Therefore, the shape of the therapeutic region 40 is designed to provide a local climate control to those areas that are generally prone to moisture accumulation. The therapeutic region 40 is in the head section 32 and seat section 37 of the patient support structure 12 as shown in Fig. 2. The large therapeutic region 40 ensures to underlie the patient's torso and pelvic region. By reducing the area of the therapeutic region 40 through which the air box 22 is required to push air, the microclimate structure 14 allows for reduction of the pressure and flow needed from an air source included in the air box 22.

The microclimate structure 14 includes a fluid flowpath having an inlet port 42. The fluid flowpath spans laterally across the microclimate structure 14 from its right side 38 to its left side 36 and extends longitudinally through the microclimate structure 14 to the head section 32 of the patient support structures 12. The inlet port 42 is directly coupled to the air box 22 via a distribution sleeve 94 and is located at the lower end 80 of the seat section 37 of the patient support structure 12. Thus, air from the air box 22 is introduced into the microclimate structure 14 at the origination point or inlet port 42 near the pelvic region of the patient lying on the microclimate structure 14. By directing the location of air introduction from the air box 22 closer to the therapeutic region 40, the microclimate structure 14 will provide an effective amount of cooling and drying to a patient's skin at the specific targeted areas, and achieve the effective result with minimal air. Having the inlet port 42 near the therapeutic region 40 prevents air from diffusing out of the microclimate structure 14 while the air flows from the inlet port 42 to the therapeutic region 40, thus requiring less volume of air. However, in some embodiments, the inlet port 42 may be positioned at the foot end of the microclimate structure 14. Further, the microclimate structure 14 may have an outlet at the head section 32 of the patient support structure 12 to exhaust the air and/or liquid. Other inlet port and outlet designs may be used in other embodiments. When the outlet is omitted, the air that traverses the microclimate structure 14 is pushed out through the perforations 41 in the therapeutic region 40 and escapes through an outer ticking layer 24 of the patient support 12. The perforations 41 include head section perforations 100 and seat section perforations 102.

An outer ticking layer 24 encompasses the microclimate structure 14 as shown in Fig. 3. The outer ticking layer 24 includes an upper ticking layer 50 and a lower ticking layer 52. The upper ticking layer 50 covers the microclimate structure 14 and the lower ticking layer 52 encases the cushion layer 16. The upper ticking layer 50 includes a breathable material that is vapor permeable but liquid impermeable. This allows the patient heat and moisture to flow away from the patient's skin in form of vapor and pass through the upper ticking layer 50 into the area which encloses the microclimate structure 14. The vapor then condenses between the upper ticking layer 50 and a first or upper layer 26 of the microclimate structure 14. At least a portion of the upper layer 26 includes a vapor and liquid permeable material which defines the therapeutic region 40. In the illustrative embodiment, the therapeutic region 40 of the upper layer 26 includes a number of perforations 41 that allows the condensed moisture and liquid from the therapeutic region 40 to flow through the upper layer 26 into a middle layer 28 of the microclimate structure 14. The upper layer 26 includes a vapor permeable but liquid impermeable material to allow vapor to flow through the upper layer 26. In the illustrated embodiments, the microclimate structure 14 and the cushion layer 16 are separated by a middle ticking layer 54, which is a top layer of the lower ticking layer 52. However, in some embodiments, a unitary outer ticking layer 24 may encase the entire patient support structure 12, including the microclimate structure 14 and the cushion layer 16.

The material of the middle layer 28 is a three-dimensional material. The three-dimensional material is arranged to extend from the upper end of the head section 32 to the lower end of the foot section 34 of the patient support structure 12 as shown in Fig. 3. The three-dimensional material is air and liquid permeable. The inlet port 42 is coupled to the lower end 80 of the seat section 37 of the three-dimensional material to allow air from the air box 22 to flow between the upper layer 26 and a lower layer 30 of the microclimate structure 14 and from the lower end 80 of the seat section 37 to the head section 32 of the patient support structure 12. Therefore, once the moisture and liquid reach the middle layer 28 from the upper layer 26, the moisture and liquid are carried away and evaporated by air flowing through the middle layer 28. The cooled-vapor can then be either directed toward the outlet or back toward the support surface 23 to cool and dry the patient's skin around the interface of the patient's skin with the support surface 23. The cooled-vapor directed toward the support surface 23 passes through the head section perforations 100 and the seat section perforations 102.

Lastly, the lower layer 30 of the microclimate structure 14 includes a liquid impermeable material to prevent liquid from leaking through the lower layer 30 into the cushion layer 16. Illustratively, the cushion layer 16 includes the inflatable support bladders 48 to support the microclimate structure 14 as shown in Fig. 3. Accordingly, the air box 22 is coupled to the microclimate structure 14 and the inflatable support bladders 48 to provide pressurized air to the support surface 23 and the cushion layer 16. In other embodiments, the cushion layer 16 may omit some or all of the inflatable support bladders 48 and utilize foam cushioning structures instead of the inflatable support bladders 48.

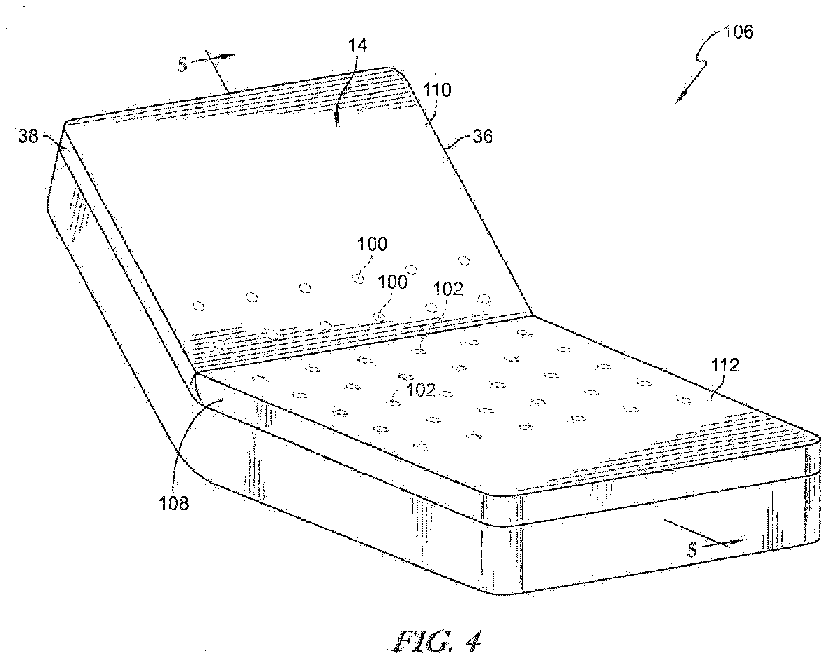

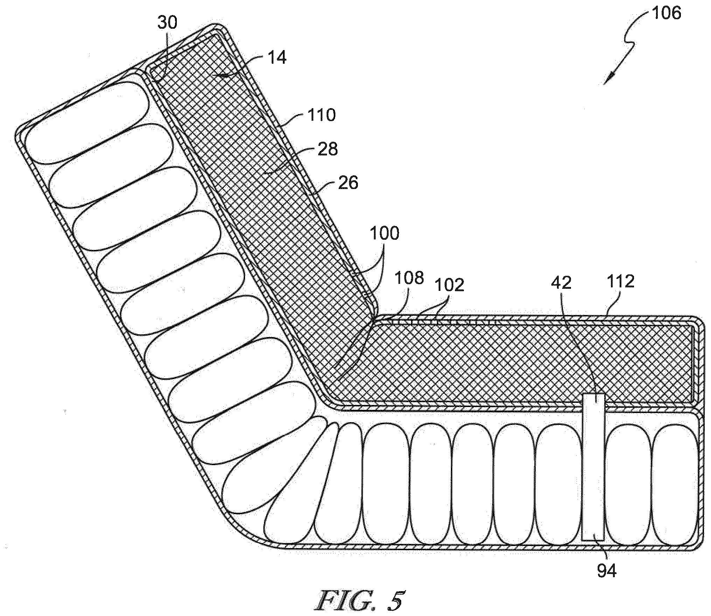

The patient support apparatus 10 is moveable between a declined position 104, shown in Figs. 2 and 3, and an inclined position 106, shown in Figs. 4 and 5. The patient support apparatus 10 moves between the declined position 104 and the inclined position 106 by altering an angle of the head section 32 relative to the seat section 37. In the declined position 104, the head section 32 extends substantially planar with the seat section 37 so that a patient may lie supine on the patient support structure 12. In the inclined position 106, the head section 32 is angled relative to the seat section 37 so that the patient may sit up in the patient support structure 12, thereby applying pressure to the seat section 37. When the patient support apparatus 10 is moved between the declined position 104 and the inclined position 106, the patient support structure 12 and the microclimate structure 14 also move between a declined and inclined position. When moved to the inclined position, the inflatable support bladders 48 may maintain an air pressure therein. Alternatively or in addition to, at least some of the inflatable support bladders 48 may deflate or inflate to accommodate the change in position of the patient support structure 12 between the declined position and the inclined position.

In the declined position 104, air flows from the distribution sleeve 94 into the microclimate structure 14 through inlet 42. The moisture and liquid in the middle layer 28 are carried away and evaporated by the air flowing through the middle layer 28. The cooled-vapor can then be either directed toward the outlet or back toward the support surface 23 to cool and dry the patient's skin around the interface of the patient's skin with the support surface 23. The cooled-vapor directed toward the support surface 23 passes through both the head section perforations 100 and the seat section perforations 102.

Referring to Figs. 4 and 5, when in the inclined positon 106, a crease 108 is formed in the microclimate structure 14. The crease 108 is formed between a head section 110 of the microclimate structure 14 and a seat section 112 of the microclimate structure 14. The crease 108 spans laterally across the microclimate structure 14 from its right side 38 to its left side 36. The crease 108 is positioned between the head section perforations 100 and the seat section perforations 102. The crease 108 extends from the upper layer 26 of the microclimate structure 14 to the lower layer 30 of the microclimate structure 14 through the middle layer 28. The crease 108 limits the three-dimensional space within the middle layer 28.

The middle layer 28 may have additional material properties in the location of the crease 108. Such additional material properties facilitate forming the crease 108 and preventing airflow through the middle layer 28. In some embodiments, the middle layer28 includes a crease material, e.g. a rubber material, a plastic material, a foam, etc., in the location where the crease 108 is formed. Alternatively, or in addition to, the middle layer 28 may have a higher density in the location where the crease 108 is formed. The additional and/or thicker material facilitates forming the crease 108 to pinch the middle layer 28, thereby limiting the airflow through the crease 108. In some embodiments, the middle layer 28 has less material or a lower density at the location where the crease 108 is formed. The reduction in material allows the ticking layer around the middle layer 28 to fold over, thereby creating the crease 108.

In some embodiments, the crease 108 extends entirely from the upper layer 26 to the lower layer 28. In such an embodiment, air flow is substantially prevented from passing through the crease 108. That is, air flow from the distribution sleeve 94 passes from inlet 42 into the seat section 112 of the microclimate structure 14, but is substantially prevented from entering the head section 110 of the microclimate structure 14. As such, the cooled-vapor formed in the middle section 28 of the microclimate structure 14 is directed toward the support surface 23 and passes through the seat section perforations 102, while being substantially prevented from passing through the head section perforations 100.

In some embodiments, the crease 108 extends partially from the upper layer 26 to the lower layer 28. In such an embodiment, air flow is limited from passing through the crease 108. The amount that air flow is limited may be determined by a size of the crease 108, i.e. how far the crease 108 extends from the upper layer 26 to the lower layer 28. Air flow from the distribution sleeve 94 passes into a seat section 112 of the microclimate structure 14 and is limited from entering the head section 110 of the microclimate structure 14, i.e. air flow into the head section 110 is starved. As such, the cooled-vapor formed in the middle section 28 of the microclimate structure 14 is directed toward the support surface 23 and passes through the seat section perforations 102, while being limited from passing through the head section perforations 100.

Although this disclosure refers to specific embodiments, it will be understood by those skilled in the art that various changes in form and detail may be made.

## Claims

1. A patient support apparatus (10) comprising:
a frame (18),
an air box (22), and
a patient support structure (12) being supported by the frame (18) and including a head section (32), a foot section (34), and a seat section (37) between the head section (32) and foot section (34), the patient support structure (12) further comprising:
a cushion layer (16),
an outer ticking layer (24) including an upper surface portion positioned to support a patient,
a microclimate structure (14) positioned within the outer ticking layer (24) and between the cushion layer (16) and the upper surface portion, the microclimate structure (14) comprising an upper layer (26), at least a portion of the upper layer (26) being vapor and liquid permeable, a middle layer (28) being air permeable, and a lower layer (30) being liquid impermeable,
a first plurality of perforations (102) extending through the upper layer (26) of the microclimate structure (14) in the seat section (37) of the patient support structure (12), and
a second plurality of perforations (100) extending through the upper layer (26) of the microclimate structure (14) in the head section (32) of the patient support structure (12),
wherein, when the patient support structure (12) is in a first position, the air box (22) supplies air flow through the first plurality of perforations (102) and the second plurality of perforations (100), and
wherein when the patient support structure (12) is in a second position, the air box (22) supplies air flow through the first plurality of perforations (102) and air flow through the second plurality of perforations (100) is limited relative to the air flow through the first plurality of perforations (102) and relative to the air flow through the second plurality of perforations (100) when the patient support structure (12) is in the first position,
wherein when the patient support structure (12) is in the second position, a crease (108) is formed in the microclimate structure (14) between the first plurality of perforations (102) and the second plurality of perforations (100), and the air flow through the second plurality of perforations (100) is limited by the crease (108),
wherein the middle layer (28) has a higher density or a lower density where the crease is formed, so as to facilitate forming of the crease (108) when the patient support structure (12) is moved to the second position.

2. The patient support apparatus (10) of claim 1, further comprising a crease material in the middle layer (28) where the crease (108) is formed.

3. The patient support apparatus (10) of claim 2, wherein the crease material comprises at least one of a rubber material, a plastic material, or a foam.

4. The patient support apparatus (10) of any preceding claim, wherein, when in the first position, the head section (32) is declined and/or when in the second position, the head section (32) is inclined.

5. The patient support apparatus (10) of any preceding claim, wherein the microclimate structure (14) extends from a lower end of the head section (32) to a lower end of the seat section (37) of the patient support structure (12), excluding the foot section (34) of the patient support structure (12).

6. The patient support apparatus (10) of any preceding claim, wherein the air box (22) is further coupled to a conduit to conduct pressurized air through the microclimate structure (14).

7. The patient support apparatus (10) of any preceding claim, wherein the vapor and liquid permeable portion of the upper layer (26) of the microclimate structure (14) defines a therapeutic region (40).

8. The patient support apparatus (10) of claim 7, wherein the therapeutic region (40) of the upper layer (26) of the microclimate structure (14) comprises a highly breathable, vapor and liquid permeable material.

9. The patient support apparatus (10) of either claim 7 or claim 8, wherein a non-therapeutic region of the upper layer (26) of the microclimate structure (14) comprises a vapor permeable but liquid impermeable material.

10. The patient support apparatus (10) of any preceding claim, wherein the middle layer (28) of the microclimate structure (14) comprises a three-dimensional material configured to conduct air between the upper layer (26) and the lower layer (30) of the microclimate structure (14).

11. The patient support apparatus (10) of claim 10, wherein the middle layer (28) of the microclimate structure (14) comprises more than one section of the three dimensional material, in which at least one section of the three dimensional material conducts and delivers air along a therapeutic region (40).

12. The patient support apparatus (10) of any preceding claim, wherein the cushion layer (16) includes a first inflatable support bladder and a second inflatable support bladder (48), and an air distribution sleeve (94) extends between the first inflatable support bladder and the second inflatable support bladder (48).

13. The patient support apparatus (10) of claim 1, wherein the outer ticking layer (24) comprises a vapor permeable and liquid impermeable material.

14. The patient support apparatus (10) of claim 1, wherein the outer ticking layer (24) encases the microclimate structure (14) and optionally also the cushion layer (16).

## Patentansprüche

1. Eine Patientenlagerungsvorrichtung (10), umfassend:
einen Rahmen (18),
einen Luftkasten (22) und
eine Patientenstützstruktur (12), die vom Rahmen (18) gestützt wird und einen Kopfbereich (32), einen Fußbereich (34) und einem Sitzbereich (37) zwischen dem Kopfbereich (32) und dem Fußbereich (34) beinhaltet, wobei die Patientenstützstruktur (12) weiter umfasst:
eine Polsterschicht (16),
eine äußere Inlettschicht (24) einschließlich eines oberen Auflageteils, der zum Stützen eines Patienten positioniert ist,
eine Mikroklimastruktur (14), die innerhalb der äußeren Inlettschicht (24) und zwischen der Polsterschicht (16) und dem oberen Auflageteil positioniert ist, wobei die Mikroklimastruktur (14) eine obere Schicht (26), bei der mindestens ein Teil der oberen Schicht (26) dampf- und flüssigkeitsdurchlässig ist, eine luftdurchlässige mittlere Schicht (28) und eine flüssigkeitsundurchlässige untere Schicht (30) umfasst,
eine erste Vielzahl an Perforationen (102), die durch die obere Schicht (26) der Mikroklimastruktur (14) im Sitzbereich (37) der Patientenstützstruktur (12) verlaufen, und
eine zweite Vielzahl an Perforationen (100), die durch die obere Schicht (26) der Mikroklimastruktur (14) im Kopfbereich (32) der Patientenstützstruktur (12) verlaufen,
wobei, wenn sich die Patientenstützstruktur (12) in der ersten Position befindet, der Luftkasten (22) einen Luftstrom durch die erste Vielzahl an Perforationen (102) und die zweite Vielzahl an Perforationen (100) zuführt, und
wobei, wenn sich die Patientenstützstruktur (12) in einer zweiten Position befindet, der Luftkasten (22) einen Luftstrom durch die erste Vielzahl an Perforationen (102) zuführt und der Luftstrom durch die zweite Vielzahl an Perforationen (100) relativ zum Luftstrom durch die erste Vielzahl an Perforationen (102) und relativ zum Luftstrom durch die zweite Vielzahl an Perforationen (100) beschränkt wird, wenn sich die Patientenstützstruktur in der ersten Position (12) befindet,
wobei, wenn sich die Patientenstützstruktur (12) in der zweiten Position befindet, eine Falte (108) in der Mikroklimastruktur (14) zwischen der ersten Vielzahl an Perforationen (102) und der zweiten Vielzahl an Perforationen (100) gebildet wird, und der Luftstrom durch die zweite Vielzahl an Perforationen (100) durch die Falte (108) beschränkt wird,
wobei die mittlere Schicht (28), wo die Falte gebildet wird, eine höhere Dichte oder eine geringere Dichte aufweist, um die Bildung der Falte (108) zu erleichtern, wenn die Patientenstützstruktur (12) in die zweite Position bewegt wird.

2. Die Patientenlagerungsvorrichtung (10) nach Anspruch 1, weiter umfassend ein Faltenmaterial in der mittleren Schicht (28), wo die Falte (108) gebildet wird.

3. Die Patientenlagerungsvorrichtung (10) nach Anspruch 2, wobei das Faltenmaterial mindestens eines von einem Gummimaterial, einem Kunststoffmaterial oder einem Schaumstoff umfasst.

4. Die Patientenlagerungsvorrichtung (10) nach einem der vorstehenden Ansprüche, wobei der Kopfbereich (32), wenn er sich in der ersten Position befindet, abgesenkt ist und/oder der Kopfbereich (32), wenn er sich in der zweiten Position befindet, schräg gestellt ist.

5. Die Patientenlagerungsvorrichtung (10) nach einem der vorstehenden Ansprüche, wobei die Mikroklimastruktur (14) von einem unteren Ende des Kopfbereichs (32) zu einem unteren Ende des Sitzbereichs (37) der Patientenstützstruktur (12) verläuft, wobei der Fußbereich (34) der Patientenstützstruktur (12) ausgeschlossen ist.

6. Die Patientenlagerungsvorrichtung (10) nach einem der vorstehenden Ansprüche, wobei der Luftkasten (22) weiter an eine Leitung angeschlossen ist, um Druckluft durch die Mikroklimastruktur (14) zu leiten.

7. Die Patientenlagerungsvorrichtung (10) nach einem der vorstehenden Ansprüche, wobei der dampf- und flüssigkeitsdurchlässige Teil der oberen Schicht (26) der Mikroklimastruktur (14) einen therapeutischen Bereich (40) definiert.

8. Die Patientenlagerungsvorrichtung (10) nach Anspruch 7, wobei der therapeutische Bereich (40) der oberen Schicht (26) der Mikroklimastruktur (14) ein sehr atmungsaktives, dampf- und flüssigkeitsdurchlässiges Material umfasst.

9. Die Patientenlagerungsvorrichtung (10) nach entweder Anspruch 7 oder Anspruch 8, wobei ein nicht-therapeutischer Bereich der oberen Schicht (26) der Mikroklimastruktur (14) ein dampfdurchlässiges, aber flüssigkeitsundurchlässiges Material umfasst.

10. Die Patientenlagerungsvorrichtung (10) nach einem der vorstehenden Ansprüche, wobei die mittlere Schicht (28) der Mikroklimastruktur (14) ein dreidimensionales Material umfasst, das so konfiguriert ist, dass Luft zwischen die obere Schicht (26) und die untere Schicht (30) der Mikroklimastruktur (14) geleitet wird.

11. Die Patientenlagerungsvorrichtung (10) nach Anspruch 10, wobei die mittlere Schicht (28) der Mikroklimastruktur (14) mehr als einen Bereich des dreidimensionalen Materials umfasst, in dem mindestens ein Bereich des dreidimensionalen Materials Luft an einem therapeutischen Bereich (40) entlangleitet und an diesen abgibt.

12. Die Patientenlagerungsvorrichtung (10) nach einem der vorstehenden Ansprüche, wobei die Polsterschicht (16) eine erste aufblasbare Stützblase und eine zweite aufblasbare Stützblase (48) beinhaltet, und ein Luftverteilungsschlauch (94) zwischen der ersten aufblasbaren Stützblase und der zweiten aufblasbaren Stützblase (48) verläuft.

13. Die Patientenlagerungsvorrichtung (10) nach Anspruch 1, wobei die äußere Inlettschicht (24) ein dampfdurchlässiges und flüssigkeitsundurchlässiges Material umfasst.

14. Die Patientenlagerungsvorrichtung (10) nach Anspruch 1, wobei die äußere Inlettschicht (24) die Mikroklimastruktur (14) und gegebenenfalls auch die Polsterschicht (16) umhüllt.

## Revendications

1. Appareil de support de patient (10) comprenant :
un châssis (18),
un caisson d'air (22), et
une structure de support de patient (12) supportée par le châssis (18) et incluant une section tête (32), une section pieds (34) et une section siège (37) entre la section tête (32) et la section pieds (34), la structure de support de patient (12) comprenant également :
une couche de rembourrage (16),
une couche de toile extérieure (24) incluant une partie supérieure de la surface positionnée pour supporter un patient,
une structure microclimatique (14) positionnée à l'intérieur de la couche de toile extérieure (24) et entre la couche de rembourrage (16) et la partie supérieure de la surface, la structure microclimatique (14) comprenant une couche supérieure (26), au moins une partie de la couche supérieure (26) étant perméable à la vapeur et aux liquides, une couche intermédiaire (28) étant perméable à l'air et une couche inférieure (30) étant imperméable aux liquides,
une première pluralité de perforations (102) s'étendant à travers la couche supérieure (26) de la structure microclimatique (14) dans la section siège (37) de la structure de support de patient (12), et
une seconde pluralité de perforations (100) s'étendant à travers la couche supérieure (26) de la structure microclimatique (14) dans la section tête (32) de la structure de support de patient (12),
dans lequel, lorsque la structure de support de patient (12) est dans une première position, le caisson d'air (22) fournit un débit d'air à travers la première pluralité de perforations (102) et la seconde pluralité de perforations (100), et
dans lequel, lorsque la structure de support de patient (12) est dans une seconde position, le caisson d'air (22) fournit un débit d'air à travers la première pluralité de perforations (102) et un débit d'air à travers la seconde pluralité de perforations (100) est limité par rapport au débit d'air à travers la première pluralité de perforations (102) et par rapport au débit d'air à travers la seconde pluralité de perforations (100) lorsque la structure de support de patient (12) est dans la première position,
dans lequel, lorsque la structure de support de patient (12) est dans la seconde position, un pli (108) est formé dans la structure microclimatique (14) entre la première pluralité de perforations (102) et la seconde pluralité de perforations (100), et le débit d'air à travers la seconde pluralité de perforations (100) est limité par le pli (108),
dans lequel la couche intermédiaire (28) a une densité supérieure ou une densité inférieure où le pli est formé, afin de faciliter la formation du pli (108) lorsque la structure de support de patient (12) est déplacée vers la seconde position.

2. Appareil de support de patient (10) selon la revendication 1, comprenant en outre un matériau de pli dans la couche intermédiaire (28) où le pli (108) se forme.

3. Appareil de support de patient (10) selon la revendication 2, dans lequel le matériau de pli comprend au moins un matériau parmi un matériau en caoutchouc, un matériau en plastique ou une mousse.

4. Appareil de support de patient (10) selon l'une quelconque des revendications précédentes, dans lequel, lorsqu'il est dans la première position, la section tête (32) est baissée et/ou lorsqu'il est dans la seconde position, la section tête (32) est inclinée.

5. Appareil de support de patient (10) selon l'une quelconque des revendications précédentes, dans lequel la structure microclimatique (14) s'étend d'une extrémité inférieure de la section tête (32) à une extrémité inférieure de la section siège (37) de la structure de support de patient (12), à l'exclusion de la section pieds (34) de la structure de support de patient (12).

6. Appareil de support de patient (10) selon l'une quelconque des revendications précédentes, dans lequel le caisson d'air (22) est en outre couplé à un conduit pour conduire l'air pressurisé à travers la structure microclimatique (14).

7. Appareil de support de patient (10) selon l'une quelconque des revendications précédentes, dans lequel la partie perméable à la vapeur et aux liquides de la couche supérieure (26) de la structure microclimatique (14) définit une région thérapeutique (40).

8. Appareil de support de patient (10) selon la revendication 7, dans lequel la région thérapeutique (40) de la couche supérieure (26) de la structure microclimatique (14) comprend un matériau hautement respirant, perméable à la vapeur et aux liquides.

9. Appareil de support de patient (10) selon la revendication 7 ou la revendication 8, dans lequel une région non thérapeutique de la couche supérieure (26) de la structure microclimatique (14) comprend un matériau perméable à la vapeur mais imperméable aux liquides.

10. Appareil de support de patient (10) selon l'une quelconque des revendications précédentes, dans lequel la couche intermédiaire (28) de la structure microclimatique (14) comprend un matériau tridimensionnel conçu pour conduire l'air entre la couche supérieure (26) et la couche inférieure (30) de la structure microclimatique (14).

11. Appareil de support de patient (10) selon la revendication 10, dans lequel la couche intermédiaire (28) de la structure microclimatique (14) comprend plus d'une section du matériau tridimensionnel, au moins une section du matériau tridimensionnel conduisant et délivrant de l'air le long d'une région thérapeutique (40).

12. Appareil de support de patient (10) selon l'une quelconque des revendications précédentes, dans lequel la couche de rembourrage (16) inclut une première poche de support gonflable et une seconde poche de support gonflable (48), et une gaine de distribution d'air (94) s'étend entre la première poche de support gonflable et la seconde poche de support gonflable (48).

13. Appareil de support de patient (10) selon la revendication 1, dans lequel la couche de toile extérieure (24) est composée d'un matériau imperméable aux liquides et perméable à la vapeur.

14. Appareil de support de patient (10) selon la revendication 1, dans lequel la couche de toile extérieure (24) recouvre la structure microclimatique (14) et éventuellement la couche de rembourrage (16).
